# EUROPEAN PATENT APPLICATION

(11) **EP 4 843 476 A1**
(43) Date of publication of application: **25.11.2026**
(21) Application number: 25177366.9
(22) Date of filing: 19.05.2025
(51) Int. Cl.: A61B 3/00, A61B 3/10, A61B 3/12

(54) **OPHTHALMIC IMAGING INSTRUMENT FOR IMAGING THE ANTERIOR AND POSTERIOR SEGMENTS OF AN EYE**

(71) Applicant: Optos plc, Dunfermline, Scotland KY11 8GR (GB)
(72) Inventor: Evans, David, Dunfermline, Scotland, KY11 8GR (GB); Bustin, Nicholas, Dunfermline, Scotland, KY11 8GR (GB); Muyo, Gonzalo, Dunfermline, Scotland, KY11 8GR (GB); Kasai, Hiroshi, Dunfermline, Scotland, KY11 8GR (GB); Mizuta, Masahiro, Minato-ku, Tokyo, 108-6290 (JP)
(74) Representative: Hoffmann Eitle

(57) **Abstract**

An ophthalmic imaging instrument operable to image, via an objective thereof, a fundus of an eye in a first mode, and an anterior segment of the eye in a second mode, the objective having a focal length adjustable between first focal length for imaging the fundus and a second focal length for imaging the anterior segment. The instrument is further operable to: project a second light onto the fundus via the objective in the first and second mode, with a degree of focus of the second light on the fundus that is variable; and focus the second light on the fundus, based on one of a degree of myopia and a degree of hyperopia of the eye, when the focal length of the objective has been adjusted to the second focal length.

## Description

### [Field]

Example aspects herein generally relate to the field of ophthalmic imaging instruments and, more particularly, to ophthalmic imaging instruments for imaging both the anterior segment and posterior segment of subjects' eyes whilst projecting light onto the ocular fundus in both cases for other purposes, such as gaze fixation or eye movement tracking, and the control of such ophthalmic imaging instruments.

### [Background]

There are various ophthalmic imaging instruments that are capable of imaging both the anterior segment (AS) and the posterior segment (PS) of the eye in a variety of imaging modalities. For example, some commercially available ophthalmic imaging instruments can be configured to allow optical coherence tomography (OCT) imaging of both the posterior segment and the anterior segment of an eye, one after another. These instruments typically employ an insertion lens for anterior segment imaging, which is inserted in front of the objective lens system for imaging the anterior segment (in a so-called "AS imaging mode"), and removed for imaging the posterior segment (in a so-called "PS imaging mode"). To allow for varying degrees of myopia (shortsightedness) and/or hyperopia (longsightedness) among subjects (these types of refractive error collectively being referred to herein as distance refractive errors), the instruments usually have an internal focus adjustment mechanism that controls the objective lens system to correct for a wide range of such refractive errors, for example from -20D to +20D (dioptres), and thus allow an in-focus image of the retina to be acquired reliably in the PS imaging mode. The focus adjustment mechanism may automatically adjust the objective lens system to acquire the in-focus image of the retina, or it may do so in accordance with instructions provided by an operator of the ophthalmic imaging instrument.

During the imaging of the posterior and anterior segments by such instruments, attempts are normally made to keep the gaze direction of the eye steady, by presenting the subject with a fixation target to fixate on via the objective lens system. Controlling the objective lens system to compensate for the subject's distance refractive error in the PS imaging mode, and thus allow in-focus images of the retina to be acquired, also tends to bring the fixation target into sharper focus for the subject, and gaze fixation is usually effective.

However, when such instruments are used to image the anterior segment of the eye, the insertion lens causes a blurred fixation target to be presented to the subject, as the objective lens system with the insertion lens in place is configured to focus on the anterior segment rather than the posterior segment, with the projection of the fixation target on the subject's retina consequently being defocused. The blurry nature of the fixation target can make it difficult for the subject to effectively fix their gaze direction in the AS imaging mode, and can therefore make the acquisition of clear AS images more challenging.

### [Summary]

There is provided, in accordance with a first example aspect herein, a controller arranged to generate control signals for controlling an ophthalmic imaging instrument which is operable in a posterior segment imaging mode to image a fundus of an eye and in an anterior segment imaging mode to image an anterior segment of the eye. The ophthalmic imaging instrument comprises an objective lens system, and an optical imaging system comprising a light source and an image-forming device, the optical imaging system and the objective lens system being arranged such that: in the posterior segment imaging mode, the light source illuminates a portion of the fundus with a first light via the objective lens system, and the image-forming device images the portion of the fundus via the objective lens system; and in the anterior segment imaging mode, the light source illuminates a portion of the anterior segment of the eye with the first light via the objective lens system, and the image-forming device images the portion of the anterior segment via the objective lens system, wherein the objective lens system has a focal length which is adjustable over focal lengths including a first focal length for imaging the portion of the fundus in the posterior segment imaging mode, and a second focal length for imaging the portion of the anterior segment in the anterior segment imaging mode. The ophthalmic imaging instrument further comprises an auxiliary optical system, which is arranged to project a second light onto the fundus via the objective lens system in the posterior segment imaging mode and in the anterior segment imaging mode, the auxiliary optical system comprising a focussing mechanism which is controllable to vary a degree of focus of the second light on the fundus. The controller comprises computer hardware arranged to: acquire one of a first indication of a degree of myopia of the eye or a second indication of a degree of hyperopia of the eye; generate a first control signal for controlling the objective lens system to set its focal length to the second focal length; and use the one of the first indication or the second indication to generate a second control signal for controlling the focussing mechanism to focus the second light on the fundus via the objective lens system having its focal length set to the second focal length (i.e. via the objective lens system when the focal length of the objective lens system is set to the second focal length for imaging the portion of the anterior segment).

In some example embodiments, the computer hardware may be arranged to: acquire the one of the first indication or the second indication by determining an amount of adjustment of the focal length of the objective lens system required to reach the first focal length for imaging the portion of the fundus in the posterior segment imaging mode; and use the determined amount of adjustment to generate the second control signal. In these or some other example embodiments, the objective lens system may comprise: a first focussing optical element and a first drive mechanism arranged to move the first focussing optical element into and out of an optical path through the objective lens system; and a second drive mechanism and a second focussing optical element whose position along the optical path through the objective lens system is adjustable by the second drive mechanism. In this case, the computer hardware is arranged to generate the first control signal for controlling the objective lens system to adjust its focal length to the second focal length by: generating a first drive control signal for controlling the first drive mechanism to move the first focussing optical element into the optical path through the objective lens system; and generating a second drive control signal for controlling the second drive mechanism to move the second focussing optical element to a position along the optical path through the objective lens system such that the focal length of the objective lens system is the second focal length for imaging the anterior segment of the eye.

There is provided, in accordance with a second example aspect herein, an ophthalmic imaging instrument which is operable in a posterior segment imaging mode to image a fundus of an eye and in an anterior segment imaging mode to image an anterior segment of the eye. The ophthalmic imaging instrument comprises an objective lens system, and an optical imaging system comprising a light source and an image-forming device, the optical imaging system and the objective lens system being arranged such that: in the posterior segment imaging mode, the light source illuminates a portion of the fundus with a first light via the objective lens system, and the image-forming device images the portion of the fundus via the objective lens system; and in the anterior segment imaging mode, the light source illuminates a portion of the anterior segment of the eye with the first light via the objective lens system, and the image-forming device images the portion of the anterior segment via the objective lens system, wherein the objective lens system has a focal length which is adjustable over focal lengths including a first focal length for imaging the portion of the fundus in the posterior segment imaging mode, and a second focal length for imaging the portion of the anterior segment in the anterior segment imaging mode. The ophthalmic imaging instrument further comprises an auxiliary optical system, which is arranged to project a second light onto the fundus via the objective lens system in the posterior segment imaging mode and in the anterior segment imaging mode, the auxiliary optical system comprising a focussing mechanism which is controllable to vary a degree of focus of the second light on the fundus. The ophthalmic imaging instrument further comprises a controller according to the first example aspect or any of its example embodiments set out above.

There is provided, in accordance with a third example aspect herein, an ophthalmic imaging instrument which is operable in a posterior segment imaging mode to image a fundus of an eye and in an anterior segment imaging mode to image an anterior segment of the eye. The ophthalmic imaging instrument comprises an objective lens system, and an optical imaging system comprising a light source and an image-forming device, the optical imaging system and the objective lens system being arranged such that: in the posterior segment imaging mode, the light source illuminates a portion of the fundus with a first light via the objective lens system, and the image-forming device images the portion of the fundus via the objective lens system; and in the anterior segment imaging mode, the light source illuminates a portion of the anterior segment of the eye with the first light via the objective lens system, and the image-forming device images the portion of the anterior segment via the objective lens system, wherein the objective lens system has a focal length which is adjustable over focal lengths including a first focal length for imaging the portion of the fundus in the posterior segment imaging mode, and a second focal length for imaging the portion of the anterior segment in the anterior segment imaging mode. The ophthalmic imaging instrument further comprises an auxiliary optical system, which is arranged to project a second light onto the fundus via the objective lens system in the posterior segment imaging mode and in the anterior segment imaging mode, the auxiliary optical system comprising a focussing mechanism which is controllable to vary a degree of focus of the second light on the fundus. The ophthalmic imaging instrument further comprises a controller arranged to control the focussing mechanism, using one of a first indication of a degree of myopia of the eye or a second indication of a degree of hyperopia of the eye, to focus the second light on the fundus via the objective lens system when the focal length of the objective lens system is set to the second focal length for imaging the portion of the anterior segment in the anterior segment imaging mode.

In some example embodiments of the third example aspect, the controller may be arranged to acquire the one of the first indication or the second indication by determining an amount of adjustment of the focal length of the objective lens system required to set it to the first focal length for imaging the portion of the fundus in the posterior segment imaging mode, and control the focussing mechanism, using the determined amount of adjustment, to focus the second light on the fundus when the focal length of the objective lens system has been adjusted to the second focal length.

In these or other example embodiments of the third example aspect, the objective lens system may comprise: a first focussing optical element whose position along an optical path through the objective lens system is adjustable to allow the focal length of the objective lens system to be set to the first focal length for imaging the fundus in the posterior segment imaging mode; and a second focussing optical element and a mechanism arranged to move the second focussing optical element into and out of the optical path through the objective lens system such that: in the anterior segment imaging mode, the second focussing optical element is in the optical path to allow the focal length of the objective lens system to be set to the second focal length for imaging the anterior segment of the eye by adjustment of the position of the first focussing optical element along the optical path through the objective lens system; and in the posterior segment imaging mode, the second focussing optical element is out of the optical path to allow the focal length of the objective lens system to be set to the first focal length for imaging the fundus by adjustment of the position of the first focussing optical element along the optical path through the objective lens system.

In some example embodiments of the second or third example aspect, the optical imaging system may comprises an optical coherence tomography (OCT) imaging system having a sample arm along which the first light propagates to the eye via the objective lens system, and a reference arm along which a reference light from the light source propagates, and the OCT imaging system and the objective lens system may be arranged such that: in the posterior segment imaging mode, the image-forming device generates an OCT image of the portion of the fundus by detecting an interference between the reference light and a portion of the first light reflected by the portion of the fundus; and in the anterior segment imaging mode, the image-forming device generates an OCT image of the portion of the anterior segment by detecting an interference between the reference light and a portion of the first light reflected by the portion of the anterior segment. The image-forming device may be further arranged to generate OCT angiography (OCTA) data of the portion of the fundus by repeatedly detecting an interference between the reference light and the portion of the first light reflected by the portion of the fundus in the posterior segment imaging mode.

In the ophthalmic imaging instrument of the second or third example aspect, or any of their example embodiments set out above, the auxiliary optical system may comprise a fixation target light projector, which is arranged to project, as the second light, a fixation target light onto the fundus for fixing a gaze direction of the eye. Alternatively, the auxiliary optical system may comprise a fundus tracking optical system, which is arranged to generate tracking data indicative of lateral movements of the fundus relative to the ophthalmic imaging instrument by acquiring a sequence of images of a portion of the fundus illuminated by the second light, and correlating images of the sequence of images to generate the tracking data.

In the ophthalmic imaging instrument of the second or third example aspects or any of their example embodiments set out above, the focussing mechanism may comprise a focussing optical element which is movable along an optical path of the second light towards the fundus so as to vary the degree of focus on the fundus of the second light projected onto the fundus.

There is provided, in accordance with a fourth example aspect herein, a computer-implemented method of generating control signals for controlling an ophthalmic imaging instrument which is operable in a posterior segment imaging mode to image a fundus of an eye and in an anterior segment imaging mode to image an anterior segment of the eye. The ophthalmic imaging instrument comprises an objective lens system, and an optical imaging system comprising a light source and an image-forming device, the optical imaging system and the objective lens system being arranged such that: in the posterior segment imaging mode, the light source illuminates a portion of the fundus with a first light via the objective lens system, and the image-forming device images the portion of the fundus via the objective lens system; and in the anterior segment imaging mode, the light source illuminates a portion of the anterior segment of the eye with the first light via the objective lens system, and the image-forming device images the portion of the anterior segment via the objective lens system, wherein the objective lens system has a focal length which is adjustable over focal lengths including a first focal length for imaging the portion of the fundus in the posterior segment imaging mode, and a second focal length for imaging the portion of the anterior segment in the anterior segment imaging mode. The ophthalmic imaging instrument further comprises an auxiliary optical system, which is arranged to project a second light onto the fundus via the objective lens system in the posterior segment imaging mode and in the anterior segment imaging mode, the auxiliary optical system comprising a focussing mechanism which is controllable to vary a degree of focus of the second light on the fundus. The computer-implemented method comprises: acquiring one of a first indication of a degree of myopia of the eye or a second indication of a degree of hyperopia of the eye; generating a first control signal for controlling the objective lens system to set its focal length to the second focal length; and using the one of the first indication or the second indication to generate a second control signal for controlling the focussing mechanism to focus the second light on the fundus via the objective lens system having its focal length set to the second focal length (i.e. via the objective lens system when the focal length of the objective lens system is set to the second focal length for imaging the portion of the anterior segment).

In some example embodiments, the one of the first indication or the second indication may be acquired by determining an amount of adjustment of the focal length of the objective lens system required to bring it to the first focal length, and the second control signal may be generated using the determined amount of adjustment.

In the computer-implemented method of the fourth example aspect or its example embodiments set out above, the objective lens system may comprise: a first focussing optical element and a first drive mechanism arranged to move the first focussing optical element into and out of an optical path through the objective lens system; and a second drive mechanism and a second focussing optical element whose position along the optical path through the objective lens system is adjustable by the second drive mechanism. In this case, generating the first control signal for controlling the objective lens system to adjust its focal length to the second focal length may comprise: generating a first drive control signal for controlling the first drive mechanism to move the first focussing optical element into the optical path through the objective lens system; and generating a second drive control signal for controlling the second drive mechanism to move the second focussing optical element to a position along the optical path through the objective lens system such that the focal length of the objective lens system is the second focal length for imaging the anterior segment of the eye. Here and in the foregoing, each focussing optical element may comprise a lens, a Fresnel lens or a Diffractive Optical Element (DOE), for example.

There is provided, in accordance with a fifth example aspect herein, a computer program comprising computer-readable instructions that, when executed by a processor, cause the processor to perform a method according to the fourth example aspect or any of its example embodiments set out above. The computer program may be stored on a non-transitory computer-readable medium or carried by a signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ophthalmic imaging instrument according to an example embodiment herein.
Figure 2 a schematic illustration of an example implementation of the ophthalmic imaging instrument of Figure 1, which comprises a fixation target light projector 40' as an example of the auxiliary optical system 40 in Figure 1.
Figure 3 is a schematic illustration of an example implementation of the auxiliary optical system 40 of Figure 1 in the form of a fundus tracking optical system 80.
Figure 4A is a schematic illustration of the objective lens system 20 in Figure 1, in a first state for imaging the posterior segment of an eye.
Figure 4B is a schematic illustration of the objective lens system 20 in Figure 1, in a second state for imaging the anterior segment of an eye.
Figure 5 is a schematic illustrating an example implementation of the controller 50 of Figure 1 in programmable signal processing hardware.
Figure 6 is a flow diagram illustrating a method according to an example embodiment, by which the controller 50 generates control signals for adjusting the focal length of the objective lens system 20 and focussing of light by the auxiliary optical system 40.
Figure 7 is a flow diagram illustrating a process by which the controller 50 may generate control signals for controlling the objective lens system 20 to set its focal length to a focal length for imaging the anterior segment of the eye.

### [Detailed Description of Example Embodiments]

In view of the challenges in acquiring AS images that are not tainted with image artefacts resulting from the relatively poor gaze fixation that tends to arise in AS imaging by conventional ophthalmic imaging instruments for AS and PS imaging as described above, and recognising similar problems that arise in other cases where light other than that used for the AS imaging is required to be in focus on the fundus of an eye during the AS imaging (for example, where retinal movement tracking during the AS imaging is required), the present inventors have devised methods and means for controlling such instruments to at least partially address these problems. More particularly, the present inventors have devised methods and means for controlling such instruments, by which an auxiliary optical system, which is arranged to project the light onto the fundus via the instrument's objective lens system in both the PS imaging mode and in the AS imaging mode, and is controllable to vary a degree of focus of the light on the fundus, can be controlled, using an indication of a distance refractive error of the eye, to focus the light onto the fundus via the objective lens system having its focal length set for AS imaging. Information on the eye's distance refractive error, which may be acquired in any suitable way, as described herein below, may thus be used to efficiently focus the light onto the fundus in preparation for the AS imaging.

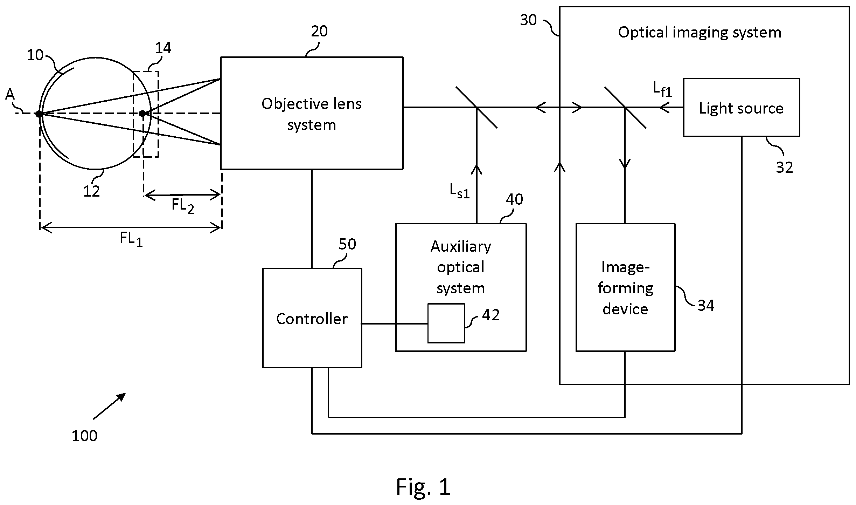

Figure 1 is a schematic illustration of an ophthalmic imaging instrument 100 according to an example embodiment. The ophthalmic imaging instrument 100 is an optical imaging instrument, which is operable in a posterior segment (PS) imaging mode to image a portion of a fundus 10 of an eye 12 of a subject. The ocular fundus 12 is the inner lining of the eye 12 opposite the lens of the eye 12, and comprises the retina, macula, optic disc, fovea, choroid, and blood vessels. The ophthalmic imaging instrument 100 is further operable in an anterior segment (AS) imaging mode to image at least a portion of an anterior segment 14 of the eye 12, the anterior segment 14 comprising the cornea, the angle, the lens, the ciliary body and the iris of the eye 12.

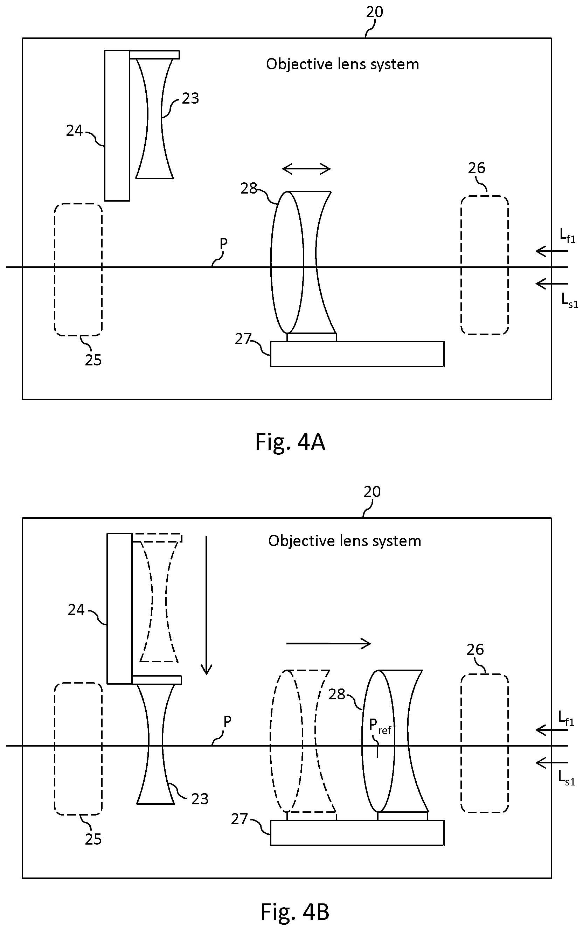

The ophthalmic imaging instrument 100 comprises an objective lens system (or objective) 20 comprising two or more lenses, and an optical imaging system 30 having a light source 32 and an image-forming device 34. The optical imaging system 30 and the objective lens system 20 are arranged such that, in the PS imaging mode, the light source 32 illuminates a portion of the fundus 10 with a first light L_{f1} via the objective lens system 20, and the image-forming device 34 images the illuminated portion of the fundus 10 via the objective lens system 20. The optical imaging system 30 and the objective lens system 20 are further arranged such that, in the AS imaging mode, the light source 32 illuminates a portion of the anterior segment 14 of the eye 12 with the first light L_{f1} via the objective lens system 20, and the image-forming device 34 images the portion of the anterior segment 14 via the objective lens system 20. To enable the imaging, in turn, of the posterior segment (which includes the fundus 10) and the anterior segment 14, the objective lens system 20 has a focal length which is adjustable over at least one range of focal lengths including a first focal length FL₁ for imaging the portion of the fundus 10 (but not the anterior segment 14) in the PS imaging mode, and a second focal length FL₂ for imaging the portion of the anterior segment 14 (but not the fundus 10) in the AS imaging mode. The focal length may, for example, be adjustable by translation of at least one lens of the objective lens system 20 along an optical axis A of the objective lens system 20, and optionally also by movement of one of more other lenses into and out of an optical path through the objective lens system 20, as described in more detail below with reference to Figures 4A and 4B.

The optical imaging system 30 may take a variety of different forms, depending on the imaging modality (or available modalities) of the ophthalmic imaging instrument 100.

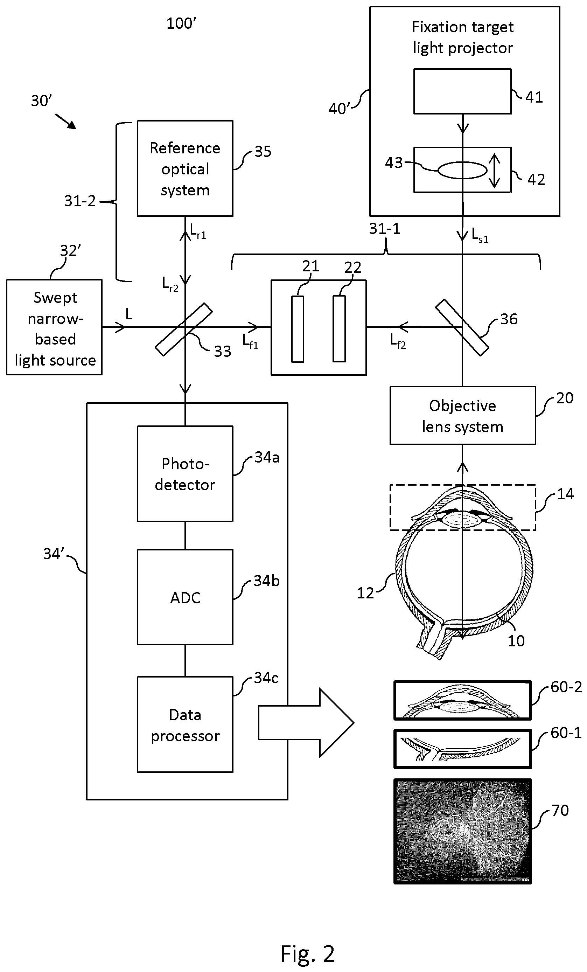

By way of an example, Figure 2 illustrates an example implementation of the ophthalmic imaging instrument 100, in for the form of an optical coherence tomography (OCT) imaging instrument 100', which comprises an implementation of the optical imaging system 30 of Figure 1 in the form of an OCT imaging system 30'. For clarity, the controller 50 is not shown in Figure 2. The OCT imaging system 30' has an interferometer with a sample arm 31-1, along which the first light L_{f1} propagates to the eye 12 via the objective lens system 20, and a reference arm 31-2, along which a reference light Lᵣ₁, Lᵣ₂ propagates. In the example of Figure 2, the OCT imaging system 30' is a swept-source OCT (SS-OCT) system, which comprises a swept narrowband light source 32', an OCT image-forming device 34' having a photodetector 34a, an analog-to-digital converter (ADC) 34b, and a data processor 34c. The OCT imaging system 30' further comprises an optical coupler 33 and a reference optical system 35.

The optical coupler 33 receives a beam of light L, which has a varying centre frequency, from the swept narrowband light source 32'. The swept narrowband light source 32' is configured to vary the centre frequency of the narrowband light L in a repetitive manner, such as by a repeated periodic frequency sweep over a defined frequency band between a lower frequency and an upper frequency. The sweep may rise in time from low frequency to high frequency, or may fall in time from high frequency to low frequency. The sweep may also successively rise and fall in time. For example, a forward sweep from low to high frequency may be followed by a reverse sweep from high to low frequency.

The swept narrowband light source 32' may be a (wavelength-) swept laser or a swept laser diode, for example an external cavity laser, an optical parametric amplifier, a Fourier-domain mode-locking laser (FDML) or a swept vertical cavity surface-emitting laser (VCSEL), although these examples are not limiting. The bandwidth of the swept narrowband light source 32' may be selected for optimum penetration through, for example, a lens of the eye 12 under examination and tissues of a retina of the eye 12. Also, the bandwidth may, as in the present example embodiment, be in the infra-red region of the optical spectrum, for example, at wavelengths longer than 850 nm.

The beam of narrowband light L generated by the swept narrowband light source 32' is directed to the optical coupler 33, which may, as in the present example embodiment, be provided in the form of a beam splitter. The beam splitter splits the beam of narrowband light L into two beams. A first of the beams propagates to the sample arm 31-1 of the interferometer, while the second beam propagates to the reference arm 31-2 of the interferometer. The splitting of the light beam L may be symmetric, such that equal intensity is directed into the resulting two beams, or may be asymmetric, such that unequal intensity is directed into the resulting two beams.

A first beam, also referred to herein as a signal light (or sample light, or first light) L_{f1}, is directed to a front-end optical system of the ophthalmic imaging instrument 100, which has the objective lens system 20 and may include further optical components arranged to shape and direct the first light L_{f1} to the eye 12 of the subject, and to collect reflected signal light L_{f2} (i.e. a portion of the signal light L_{f1} reflected from the eye 12) and return it to the optical coupler 33. The reflected signal light L_{f2} follows essentially the same path to the optical coupler 33 as the signal light L_{f1}, but in a reverse direction.

The front-end optical system may include conventional components known in the field of optical coherence tomography, and such conventional components may be adapted according to the imaging operation to be performed. More particularly, the front-end optical system may, as in the present example embodiment, include scanning optics which can cause a pivoting of the signal light L_{f1} about a pivot point located in the anterior segment 14 of the eye 12 in order to scan the signal light L_{f1} across a wide field of the fundus 10 during operation in the PS imaging mode.

The ophthalmic imaging instrument 100 may, as in the present example embodiment, be a widefield (WF) ophthalmic imaging instrument, wherein the objective lens system 20 is arranged to allow acquisition of a WF image of the fundus 10. A WF image of the fundus 10 is defined as a single-capture image, centred on the fovea of the eye 12, which captures retinal anatomic features in the mid-periphery that are posterior to the vortex vein ampulla, in all four quadrants (i.e. superior, inferior, nasal and temporal) of the eye 12. A WF ophthalmic imaging instrument can thus acquire a single-capture image which covers a region of the retina extending from the fovea up to the posterior edge of vortex vein ampulla, and is defined to have a field of view (FoV) between 60 and 100 degrees. Here, the FoV is expressed in terms of the eye-angle, which is the angle subtended by the imaged retinal region at the spherical centre of the eye 12, i.e. the point of intersection between the vertical diameter of the eyeball and the visual axis.

In some example embodiments, the ophthalmic imaging instrument 100 may be provided in form of an ultra-widefield (UWF) ophthalmic imaging instrument having a larger FoV than a WF instrument, wherein the objective lens system 20 is arranged to allow acquisition of an UWF image of the fundus 10 covering a larger proportion of the fundus 10. An UWF image of the fundus 10 is defined as a single-capture image, centred on the fovea of the eye 12, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so-called Optomap^{™} images) covering up to 200 degrees of the fundus (or approximately 82% of the retina) in a single capture.

It should be noted that the techniques described herein are not limited in their applicability to WF and UWF ophthalmic imaging instruments that are operable to image both the anterior and posterior segment of the eye 12 but are also applicable to ophthalmic imaging instruments for imaging the anterior segment and the posterior segment with a smaller FoV.

The front-end optical system may, as in the present example embodiment, include one or more scanners, for example a first galvanometer scanner ("H-galvo") 21 and a second galvanometer scanner ("V-galvo") 22, as shown in Figure 2, which are arranged to scan the beam of signal light L_{f1} in different (e.g. orthogonal) directions across the fundus 10. Each scanner may alternatively be micro electro-mechanical system (MEMS) scanner, a prism or polygon scanner or a resonant scanner, for example. Furthermore, where the front-end optical system has two or more scanners, the scanners may be of different kinds, e.g. one being a galvanometer scanner and another being a MEMS scanner.

Referring again to the optical coupler 33 in Figure 2, a second beam, also referred to herein as a reference light Lᵣ₁, is directed by the optical coupler 33 to the reference optical system 35, which is configured to return the reference light to the optical coupler 33. At the optical coupler 33, the returning reflected signal sample L_{f2} (i.e. the signal light L_{f1} reflected from the eye 12) and the returning reference light Lᵣ₂ (i.e. the reference light returned from the reference optical system 35) are combined to interfere with one another, and are directed as interfering beams to the photodetector 34a. The photodetector 34a may, for example, comprise a photodiode or an avalanche photodiode, which converts an optical intensity of the interfering beams to provide a resulting converted signal in the form of, e.g. an electrical signal such as a voltage or current. The resulting converted signal may, as in the present example embodiment, be a time-varying analogue signal. After being output by the photodetector 34a, the resulting converted signal may be recorded in a time-varying manner, wherein a recorded version of the signal constitutes an interferogram between the returning reflected signal light L_{f2} and the returning reference light Lᵣ₂ with respect to the optical frequency of the narrowband light from the swept narrowband light source 32'.

The ADC 34b periodically samples and quantises the signal output by the photodetector 34a with a predetermined sample frequency, and then provides digital values of the quantised and sampled signal to the data processor 34c, wherein the values are recorded. Accordingly, the photodetector 34a and the ADC 34b together constitute the detector unit which samples the time-varying interference signal. The data processor 34c may, as in the present example embodiment, comprise a processor and a memory storing computer-readable instructions that, when executed by the processor, cause the processor to perform a Fourier transform operation, such as a fast Fourier transform, on the time-varying quantised and sampled signal values obtained from the ADC 34b to generate an axial depth profile. The axial depth profile may be provided in the form of a one-, two- or three-dimensional data array, depending on whether the measurement beam is scanned across the eye 12 and, if so, the dimensionality of the scan.

As a result of the periodic sampling of the output of photodetector 34a by the ADC 34b, the highest-frequency components of the time-varying analogue signal, which lie outside a detection bandwidth of the system, will be aliased, potentially compromising the image. The bandwidth of the system is defined as a combination of electronic bandwidth, which determines the highest frequency that can be detected, and the bandwidth of the ADC 34b, which determines the range of frequencies which can be recorded uniquely (i.e. without aliasing). An anti-aliasing filter, which matches the bandwidth of the ADC 34b, may be provided between the photodetector 34a and the ADC 34b.

The swept narrowband light source 32b may have relatively high coherence length such that the interference between the returning reflected signal light L_{f2} and the returning reference light Lᵣ₂ contains information about the reflectivity of the funds 10 (or portion of the anterior segment 14 being imaged, as the case may be) over a large axial depth range defined by the high coherence length. Nonetheless, only information about a sub-range of this axial depth range corresponding to the detection bandwidth, centred on an axial position defined by the optical path length of the reference arm, is available from a Fourier transform of the sampled signal recorded at the data processor 34c.

The optical path length of the sample arm 31-1 or reference arm 31-2 may be adjusted to set an axial position of the measured axial depth range to correspond to the position of the surface under investigation, for example the fundus 10 or a portion of the anterior segment 14 that is of interest. Thereby, an optical delay between the lights the two arms of the interferometer may be adjusted, as necessary, when switching from the posterior imaging mode to the anterior imaging mode, and vice versa. As an example, a moveable reflector (not shown in Figure 2), such as a movable mirror, may be provided in the reference optical system 35 to alter the optical path length travelled by light in the reference arm 31-2. Alternatively, an arrangement of one or more corner cube reflectors (not shown in Figure 2) may be provided to alter the optical path length travelled by light in the sample arm 31-1.

The arrangement of the OCT imaging system 30' and the objective lens system 20 described above allows the OCT image-forming device 34' to generate an OCT image 60-1 of the illuminated portion of the fundus 10 by detecting an interference between the returning reference light Lᵣ₂ and returning reflected signal light L_{f2} in the PS imaging mode, and to generate an OCT image 60-2 of the illuminated portion of the anterior segment 14 by detecting an interference between the returning reference light Lᵣ₂ and returning reflected signal light L_{f2} in the AS imaging mode.

Although the example embodiments described above employ free-space optics, those arrangements are not limiting. In other example embodiments herein, optical fibre-based configurations can be employed, in which optical fibres are used in place of free space to propagate beams of light, and a fibre coupler unit (FCU) may be used in place of the beam splitter described above.

Furthermore, although the example embodiment of Figure 2 is implemented using so-called unbalanced detection, in which the interfering frequency components are directly detected from an optical signal by the photodetector 34a, a balanced detection arrangement may be employed in other example embodiments, in which the return lights from the signal arm 31-1 and the reference arm 31-2 are combined at a (e.g. 50:50) coupler and the outputs of the coupler are coupled to different optical inputs of a balanced detector. In such an arrangement, the intensity of the signal and reference light may also independently be measured, for example, by diverting at least some of each of the sample and reference light to a separate detection element, such that variations in the intensity of light can be compensated by the detection elements.

Further still, although an example of the optical imaging system 30 in the form of an SS-OCT imaging system 30' has been described above with reference to Figure 2, this is only an example, and the optical imaging system 30 may take many other forms whose implementations are well-known. The optical imaging system 30 may, for example, be another kind of Fourier-domain OCT (FD-OCT) imaging system, such as a spectral-domain OCT (SD-OCT) imaging system, or may alternatively be a time-domain OCT (TD-OCT) imaging system.

Moreover, the optical imaging system 30 is not limited to having an OCT imaging modality and may generally be an optical imaging system of any imaging modality (possibly having two or more imaging modalities) that is capable of sequentially imaging the posterior segment and the anterior segment of the eye 12. For example, the optical imaging system 30 may additionally or alternatively comprise a scanning laser ophthalmoscope (SLO), which is configured to acquire reflectance images of both the fundus 10 and the anterior segment 14 of the eye 12, for example. The SLO may, for example, be a flying spot (or point-scan) SLO, which scans a light beam across the imaging target in accordance with a scan pattern (e.g. a raster scan) whilst detecting light reflected by the imaging target to build up an image of the imaging target, or a line-scanning SLO, which may have a shorter image acquisition time.

In some example embodiments, including the present one, the optical imaging system 30 is operable to acquire fundus images of a different modality than the images of the anterior segment, as well as images of the fundus and the anterior segment 14 that are of the same modality. For example, the image-forming device 34' may, as in the present example embodiment, be further arranged to generate an OCT angiography (OCTA) image 70 of the illuminated portion of the fundus 10 by repeatedly detecting an interference signal resulting from an interference between the reference light Lᵣ₂ and the portion L_{f2} of the first light reflected by the portion of the fundus 10 in the PS imaging mode, for example by acquiring repeat B-scans of a targeted region of the fundus 10. The image-forming device 34' may then generate OCTA data representable as the OCTA image 70 by processing the detected interference signals using well-known OCTA data processing techniques, a review of which may be found in the article "Optical Coherence Tomography Angiography" by S. S. Gao et al., Investigative Ophthalmology & Visual Science, Special Issue, Vol. 57, No. 9, OCT27-OCT36, for example.

Referring again to Figure 1, the ophthalmic imaging instrument 100 further comprises an auxiliary optical system 40, which may be any optical system that is arranged to project a second light Lₛ₁ onto the fundus 10 via the objective lens system 20 during operation of the ophthalmic imaging instrument 100 in both the PS imaging mode and the AS imaging mode. The auxiliary optical system 40 comprises a focussing mechanism 42, which is controllable to vary a degree of focus of the second light Lₛ₁ on the fundus 10, i.e. to vary a location of a focal plane (or focal point) in the eye 12, onto which plane (or point) the second light Lₛ₁ is focussed by the objective lens system 20, such that an extent to which the second light Lₛ₁ is in focus on the fundus 10 is variable by the focussing mechanism 42. The focussing mechanism 42 may comprise a lens or other kind of focussing optical element 43 (e.g. a Fresnel lens or a diffractive Optical Element, DOE), which is moveable such that its optical axis moves in a direction parallel to an optical path of the second light Lₛ₁ passing through the focussing optical element 43, thus varying the degree of focus on the fundus 10 of the second light Lₛ₁ being projected onto the fundus 10 via the objective lens system 20. As an alternative, the focussing mechanism 42 may, for example, comprise a liquid lens having a refractive surface which is deformable so as to vary the degree of focus on the fundus 10 of the second light Lₛ₁ being projected onto the fundus 10. An optical coupler 36, such as a dichroic mirror or half-mirror, may be arranged to reflect the first light L_{F1} into the objective lens system 20 and allow the second light Lₛ₁ to propagate therethrough into the objective lens system 20, as illustrated in Figure 2.

The auxiliary optical system 40 may, as in the present example embodiment, be provided in the form of a fixation target light projector, which is arranged to project, as the second light Lₛ₁, a fixation target light onto the fundus 10 for fixing a gaze direction of the eye 12. Providing the subject with a fixation target to focus their gaze direction on during imaging by the ophthalmic imaging instrument 100 tends to reduce movements of the eye 12 during its imaging and therefore avoid or at least reduce image artefacts in images of the eye 12 acquired by the ophthalmic imaging instrument 100. The fixation target light projector may comprise one or more (in some cases, a matrix of) light sources 41 such as light-emitting diodes (LEDs), for example, which is/are arranged to emit visible light. In some example embodiments, the fixation target light projector may include a display screen arranged to display a fixation target. A projection on the fundus 10 of the fixation target light from the display screen or other fixation target light source 41, via the objective lens system 20, may have a predetermined shape chosen to assist the subject in maintaining a steady gaze direction, which may include one or more of a crosshair, a dot, a disc or a circle, for example.

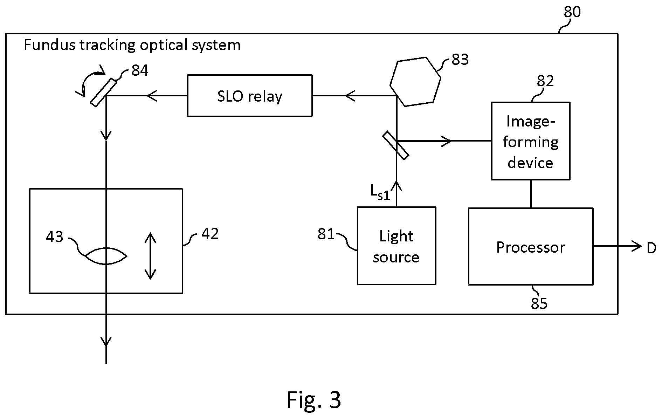

The auxiliary optical system 40 may take other forms, however. For instance, in some other embodiments, the auxiliary optical system 40 comprises a fundus tracking optical system 80, as illustrated schematically in Figure 3, which is arranged to generate tracking data D indicative of lateral movements on the fundus 10 relative to the ophthalmic imaging instrument 100 (i.e. movements of the fundus 10 that are normal to the optical axis A of the objective lens system 20). The fundus tracking optical system 80 may employ a light source 81 to repeatedly illuminate a selected portion of the fundus 10 with the second light Lₛ₁, and an image-forming device 82 to acquire an image of the portion of the fundus 10 each time it is illuminated, thus acquiring a sequence of images of the (same) illuminated portion of the fundus 10. The fundus tracking optical system 80 may, as in the example of Figure 3, acquire each image by scanning a beam of the second light Lₛ₁ using a polygon scanner 83 and a galvanometer scanner 84 (or scanners of other well-known kinds) across the portion of the fundus 10 and de-scanning the return light via the same optics. The fundus tracking optical system 80 may further comprise a processor 85 arranged to correlate images of the acquired sequence of images, for example by calculating a cross-correlation between (e.g. adjacent) images in the sequence, to acquire the tracking data D. The tracking data D may be used in a control loop which aims to maintain the position on the fundus 10 where the signal light L_{f1} is projected onto the fundus 10, by controlling the first galvanometer scanner 21 and the second galvanometer scanner 22 to compensate for the movements. Use of the tracking data D in this way may be particularly beneficial for the acquisition of OCTA images of the retina, although it may also help avoid or allow the removal of movement-related image artefacts in images of the anterior segment 14 of the eye 12.

In some example embodiments, the ophthalmic imaging instrument 100 may comprise both the fixation target light projector and the fundus tracking optical system 80 (or any of the variants of either of these devices that have been described above), and these may be coupled to the objective lens system 20 by any suitable means, such as one or more dichroic mirrors or half-mirrors, for example.

Referring to Figures 4A and 4B, the objective lens system 20 may, as in the present example embodiment, comprise a first focussing optical element 23, and a first drive mechanism 24 arranged to move the first focussing optical element 23 into and out of an optical path P (e.g. of the first light Lₛ₁) through the objective lens system 20. Put another way, the first drive mechanism 24 is arranged to move the first focussing optical element 23 (either by translation using rails or the like, as in the present example, or by rotation, for example) between a first position (as shown in Figure 4A) and a second position (as shown in Figure 4B), such that light propagating through the objective lens system 20 does not propagate through the first focussing optical element 23 when it is disposed at the first position but does propagate through the first focussing optical element 23 when it is disposed at the second position. Accordingly, the first focussing optical element 23, when disposed at the second position, contributes to a focussing of light that is performed by the objective lens system 20 and, when disposed at the first position, makes no contribution to the focussing. As will be described in more detail below, for operation in the AS imaging mode, the first drive mechanism 24 is controlled to set the first focussing optical element 23 at the second position shown in Figure 4B for imaging the anterior segment 14 of the eye 12. For operation in the PS imaging mode, the first drive mechanism 24 is controlled to set the first focussing optical element 23 at the first position so that the ophthalmic imaging instrument 100 can image the fundus 10 and/or another part of the posterior segment of the eye 12 via the objective lens system 20.

The first focussing optical element 23 may, as in the present example embodiment, comprise a lens of negative refractive power. The first focussing optical element 23 is not so limited, however, and may alternatively comprise a lens of positive refractive power, or another kind of focussing means, such as a Fresnel lens or a Diffractive Optical Element (DOE), for example.

The objective lens system 20 may, as in the present example embodiment, further comprise a first lens group 25 of one or more lenses of overall positive refractive power, and a second lens group 26 of one or more lenses likewise having an overall positive refractive power, with all of the lenses' optical axes coinciding. The first focussing optical element 23 may be provided in the optical path P between the first lens group 25 and the second lens group 26 when the first focussing optical element 23 is disposed in the second position, as illustrated in Figure 4B. The first focussing optical element 23 may alternatively be disposed on the other side of the second lens group 26 in the optical path P, specifically between the second lens group 26 and the galvanometer scanners 21 and 22, when the first focussing element 23 is in the second position.

The objective lens system 20 may, as in the present example embodiment, further comprise a second drive mechanism 27 and a second focussing optical element 28, whose position along the optical path P through the objective lens system 20 is adjustable by the second drive mechanism 27. The second focussing optical element 28 may, as in the present example embodiment, be disposed between the first lens group 25 and the second lens group 26, and may have a convex lens surface facing the first lens group 25 and a concave lens surface facing the second lens group 26, for example. As will be described in more detail below, for operation in the AS imaging mode, the second drive mechanism 27 is controlled to move the second focussing optical element 28 to a reference position P_{ref}, along the optical path P, whereby the focal length of the objective lens system 20 is set to the second focal length LF₂ for imaging the portion of the anterior segment 14 that is of interest. For operation in the PS imaging mode, the second drive mechanism 27 is controlled to move the second focussing optical element 28 in either direction along the optical path P so as to vary the focal length of the objective lens system 20, until the focal length of the objective lens system 20 is set to the first focal length FL₁ for acquiring an in-focus image of the portion of the fundus 10 that is of interest.

For further details of how the objective lens system 20 may be configured for imaging the fundus 10 and the anterior segment 14 of the eye 12 by selective insertion of the first focussing optical element 23 into the optical path P within the objective lens system, as described above, reference is made to US 2022/0079434 A1, the contents of which are incorporated herein by reference in their entirety. It should be noted, however, that the optical arrangement employed in the objective lens system 20 is not so limited, and the techniques described herein are applicable to any ophthalmic imaging instrument having an objective lens system with a focal length that is adjustable to allow imaging of the anterior segment and the posterior segment of an eye (for example, an objective lens systems having a lens attachment insertable between an objective lens and the eye under examination, as described in US 2008/0106696 A1), and an auxiliary optical system that is required to focus light on the fundus of the eye during imaging of both of the segments.

Referring again to Figure 1, the ophthalmic imaging instrument 100 also has a controller 50, which is operatively coupled to the objective lens system 20, the optical imaging system 30 and the auxiliary optical system 40.

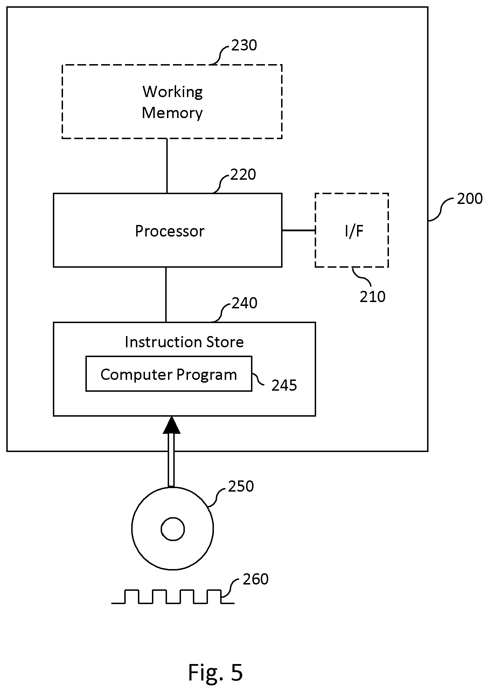

The controller 50 may be provided in any suitable form, for example as a processor 220 of a programmable signal processing hardware 200 of the kind illustrated schematically in Figure 5. The programmable signal processing hardware 200 comprises a communication interface (I/F) 210, for communicating control signals and/or data with the objective lens system 20, the optical imaging system 30 and the auxiliary optical system 40, as described herein. The signal processing hardware 200 further comprises a processor 220 (e.g. a Central Processing Unit, CPU, and/or a Graphics Processing Unit, GPU), a working memory 230 (e.g. a random-access memory) and an instruction store 240 storing a computer program 245 comprising the computer-readable instructions which, when executed by the processor 220, cause the processor 220 to perform various functions of the controller 50 described herein.

The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the controller 50 as described herein. More generally, the controller 50 of the present example embodiment may comprise one or more instances of the computer processor 220 and one or more instances of the memory 240 storing computer-readable instructions which, when executed by the computer processor(s) 220, cause the computer processor(s) 220 to perform some or all of the functions of the controller 50 as described herein. Where a plurality of processors 220 is provided, the processors 220 may communicate with each other via any kind of computer network.

It should be noted, however, that the controller 50 may alternatively be implemented in non-programmable hardware, such as an ASIC, an FPGA or other integrated circuit dedicated to performing the functions of the controller 50 described herein, or a combination of such non-programmable hardware and programmable hardware as described above with reference to Figure 5. The controller 50 may thus be implemented in any computer hardware configured to provide the described functionality.

When the ophthalmic imaging instrument 100 is to be used to image a portion of the fundus 10, the controller 50 adjusts the focal length of the objective lens system 20 to the first focal length FL₁, and sets the optical delay between the two arms of the interferometer for imaging the fundus 10. The controller 50 may adjust the focal length starting from a nominal (e.g. zero dioptre) focal length, which would be suitable for imaging the fundus of an eye of an average size which is non-myopic, for example. The nominal focal length may alternatively be suitable for imaging the fundus of an eye which has a prescribed level of myopia that is average for a population of interest, for example. The controller 50 may adjust the focal length of the objective lens system 20 from the nominal focal length to the first focal length FL₁ that is suitable for acquiring a focused image of the subject's fundus 10 (i.e. an image of the fundus 10 having a maximum degree of focus achievable) by controlling the movement of one or more lenses of the objective lens system 20 relative to a remainder of the objective lens system 20, along the optical axis of the objective lens system 20.

For example, in the implementation of the objective lens system 20 described above with reference to Figures 4A and 4B, the controller 50 generates a drive control signal to control the first drive mechanism 24 to move the first focussing optical element 23 out of the optical path P through the objective lens system 20, and controls the optical imaging system 30 to acquire images of the posterior segment of the eye 12 while controlling the second drive mechanism 27 with respective drive control signals to move the second focussing optical element 28 along the optical path P, from a starting position corresponding to the nominal focal length mentioned above, thereby adjusting the focal length of the objective lens system 20. The controller 50 may, as in the present example embodiment, analyse images acquired by the image-forming device 34 as the adjustment is being made to determine when the focal length of the objective lens system 20 has been adjusted to the first focal length FL₁ for imaging the fundus 10. For example, where the image-forming device 34 takes the form of an OCT image-forming device 34' (as in the example of Figure 2 discussed above), the controller 50 may analyse OCT images (e.g. B-scans) generated by the data processor 34c to determine the location, within an image frame of the OCT images, of a predetermined anatomical feature of the fundus 10 that is present in the OCT images, such as the surface of the retina or a prescribed retinal layer (e.g. the retinal pigment epithelium, RPE), and can be reliably detected automatically from its characteristic spatial intensity variation, for example. Once the controller 50 determines that the predetermined anatomical feature is at a predetermined location (or with a predetermined range of locations) within the image frame, the controller 50 determines that the focal length of the objective lens system 20 has been adjusted to the first focal length FL₁, and stops adjusting the focal length by movement of the second focussing optical element 28. By monitoring the drive control signals used to control the second drive mechanism 27 or by independent measurement of the displacement of the second focussing optical element 28 during the focal length adjustment, for example, the controller 50 may determine the amount of adjustment of the focal length of the objective lens system 20, from the nominal (e.g. zero dioptre) focal length, that was required to bring the focal length to the first focal length FL₁. The determined amount of adjustment provides an indication of the distance refractive error of the eye 12, specifically the degree of myopia (short-sightedness) or hyperopia/hypermetropia (longsightedness) of the eye 12, as the case may be.

As another example, where the image-forming device 34 comprises an SLO as described above, the controller 50 may be arranged to analyse the fundus images being acquired as the focal length of the objective lens system 20 is being adjusted (by the controller 50 controlling the second drive mechanism 27 with respective drive control signals to move the second focussing optical element 28 along the optical path P), to evaluate a focus metric that is indicative of the degree of focus of the images, and determine, from the values of the focus metric, when the focal length of the objective lens system 20 has been adjusted to the first focal length FL₁ for imaging the fundus 10. The focus metric may be any of the well-known kinds that are used in auto-focussing systems, for example, such as those discussed in the article titled "An extensive empirical evaluation of fucus measures for digital photography" by H. Mir and P. van Beek, Proc. SPIE 9023, Digital Photography X, 902301 (7 March 2014), the contents of which are incorporated herein by reference in their entirety. By monitoring the values of the focus metric during the focal length adjustment, and by monitoring the drive control signals used to control the second drive mechanism 27 or by independent measurement of the displacement of the second focussing optical element 28 during the focal length adjustment, for example, the controller 50 may determine the amount of adjustment of the focal length of the objective lens system 20, from the nominal (e.g. zero dioptre) focal length, that was required to bring it to the first focal length FL₁. Here again, the determined amount of adjustment provides an indication of the distance refractive error of the eye 12, specifically the degree of myopia or hyperopia of the eye 12, as the case may be.

The controller 50 need not acquire the indication of the degree of myopia/hyperopia of the eye 12 from the amount of adjustment of the focal length of the objective lens system 20 that is required to bring it to the first focal length FL₁, however, and may acquire the degree of myopia/hyperopia of the eye 12 in another way, for example from an autorefractor in example embodiments where such an instrument forms part of the ophthalmic imaging instrument 100. As a further alternative, the ophthalmic imaging instrument 100 may comprise a user interface (comprising a keyboard or a touchscreen, for example) operatively connected to the controller 50, via which user interface a user may provide the controller 50 with a measured degree of myopia/hyperopia of the eye 12 that has been measured in a visual acuity test, for example.

The ophthalmic imaging instrument 100 is then ready to image the fundus 10. For improved image quality through reduced motion artefacts, gaze fixation is preferably employed to fix the gaze direction of the eye 12 in a gaze direction that is appropriate for the target region of the fundus 10 that is to be imaged, e.g. a central gaze direction. Accordingly, in the present example embodiment, the controller 50 generates and applies to the focussing mechanism 42 a control signal to cause the focussing mechanism 42 to focus the fixation target light Lₛ₁ onto the fundus 10 when the fixation target light Lₛ₁ is projected onto the fundus 10 via the objective lens system 20, when the focal length of the objective lens system 20 is the first focal length FL₁. The control signal may appropriately set the position of the focussing optical element 43 along its adjustment direction, as described above with reference to Figure 2, or appropriately set a drive voltage applied to an electrode of the liquid lens of the focussing mechanism 42 in the alternative also described above, for example. The controller 50 preferably applies the control signal to the focussing mechanism 42, and controls the fixation target light projector to project the fixation target light Lₛ₁ onto the fundus 10 via the focussing mechanism 42 and the objective lens system 20, before generating and using the drive control signals discussed above to move the second focussing optical element 28 along the optical path P and thereby adjusting the focal length of the objective lens system 20 to the first focal length FL₁. The focal planes of the signal light L_{f1} and the fixation target light Lₛ₁ may be coincident (or nearly so) and move together whilst maintaining a fixed relative position with respect to each other during the adjustment of the focal length of the objective lens system 20 by the controller 50. The subject may therefore observe the fixation target to gradually come into focus as the focal length of the objective lens system 20 is being adjusted to the first focal length FL₁. The increase in the degree of focus of the fixation target (particularly where the fixation target is an image of a recognisable object, such as a hot air balloon, for example), for may help maintain the subject's interest in the fixation target and thus assist the subject in maintaining a steady gaze direction. With subject having a clear (in-focus) fixation target to fixate on, imaging of the fundus 10 can begin.

To acquire the OCT image 60-1 of the fundus 10, the light source 32' of the OCT imaging system 30' illuminates a portion of the fundus 10 with the first light L_{f1} via the objective lens system 20 while the focal length of the objective lens system 20 remains at the first focal length FL₁, and the image-forming device 34' images the illuminated portion of the fundus 10 as described above. OCT imaging of the fundus 10 (or OCTA imaging of the fundus, as the case may be) has then been completed, and the ophthalmic imaging instrument 100 switches from operating in the PS imaging mode to operating in the AS imaging mode.

To enable a seamless transition between these modes from the subject's perspective, the controller 50 preferably controls the objective lens system 20 and the focussing mechanism 42 to maintain a projection of the fixation target light onto the fundus 10 that is in focus on the fundus, with as little interruption as possible, and with a change in the degree of focus of the fixation target light on the fundus 10, when switching from the PS imaging mode to the AS imaging mode, which is preferably small enough to be imperceptible to the subject. This is desirable because a perceived change in the appearance of the fixation target by the subject creates a risk of distracting the subject, causing them to avert their gaze from the fixation target during the switch from the posterior imaging mode to the anterior mode, or even disengage from the ophthalmic imaging instrument 100 if the subject takes the change to signal the end of the imaging session. The following processes are performed with this aim in mind.

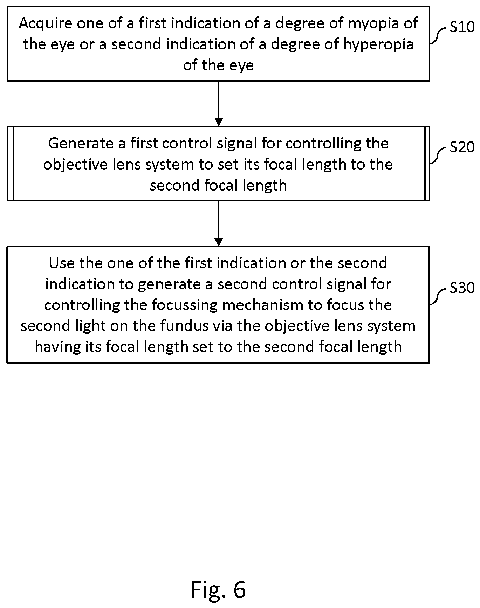

Figure 6 is a flow diagram illustrating a method according to an example embodiment, by which the controller 50 generates control signals for adjusting the focal length of the objective lens system 20 and focussing of light by the auxiliary optical system 40.

In process S10 of Figure 6, the controller 50 acquires one of a first indication of a degree of myopia of the eye 12 or a second indication of a degree of hyperopia of the eye 12. The controller may acquire such indication of the distance refractive error of the eye 12 in any of the ways described above (among others). For example, the controller 50 may determine the first indication or the second indication (as the case may be) by determining an amount of adjustment of the focal length of the objective lens system 20 required to bring it to the first focal length FL₁, for example by monitoring the drive control signals used to control the second drive mechanism 27 or by independent measurement of the displacement of the second focussing optical element 28 during the adjustment of the focal length to the first focal length FL₁. The controller 50 may determine when the focal length has reached the first focal length FL₁ during the adjustment in any of the ways described above, for example, depending on the imaging modality of the optical imaging system 30.

Before imaging of the anterior segment 14 of the eye 12 can begin, the controller 50 is required to adjust the focal length of the objective lens system 20 to the second focal length FL₂, and adjust the optical delay between the two arms of the interferometer for imaging a portion of the anterior segment 14. Accordingly, in process S20 of Figure 6, the controller 50 generates a control signal for controlling the objective lens system 20 to sets its focal length to the second focal length FL₂. The control signal is applied to the objective lens system 20, which uses the control signal to sets its focal length to the second focal length FL₂.

In the implementation of the objective lens system 20 in Figures 4A and 4B, for example, the controller 50 generates a first drive control signal for controlling the first drive mechanism 24 to move the first focussing optical element 23 into the optical path P passing through the objective lens system 20, and generates a second drive control signal for controlling the second drive mechanism 27 to move the second focussing optical element 28 to the reference position P_{ref} along the optical path P, such that the focal length of the objective lens system 20 becomes the second focal length FL₂ for imaging the anterior segment 14.

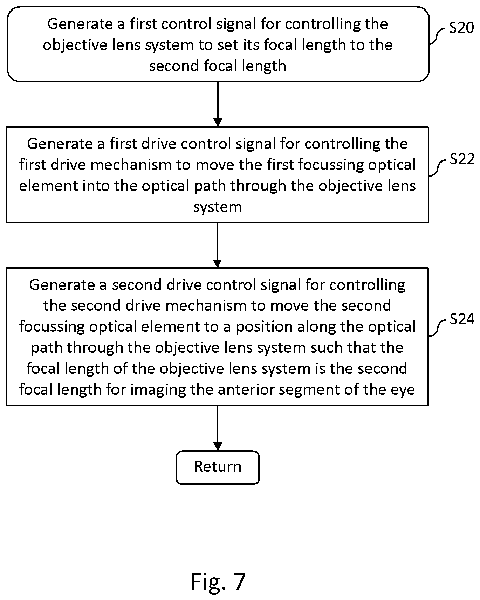

In example embodiments where the objective lens system 20 comprises a first focussing optical element 23 and a first drive mechanism 24 arranged to move the first focussing optical element 23 into and out of an optical path P through the objective lens system 20, as well as a second drive mechanism 27 and a second focussing optical element 28 whose position along the optical path P through the objective lens system 20 is adjustable by the second drive mechanism 25, as illustrated in Figures 4A and 4B, process S20 of Figure 6 may comprise generating (in process S22 of Figure 7), a first drive control signal for controlling the first drive mechanism 24 to move the first focussing optical element 23 into the optical path P through the objective lens system 20. Process 20 of Figure 6 may furthermore comprise generating (in process S24 of Figure 7) a second drive control signal for controlling the second drive mechanism 27 to move the second focussing optical element 28 to a position along the optical path P through the objective lens system 20 such that the focal length of the objective lens system 20 becomes the second focal length FL₂ for imaging the anterior segment 14 of the eye 12.

Preferably simultaneously with the adjustment of the focal length of the objective lens system 20 to the second focal length FL₂, in process S30 of Figure 6, the controller 50 uses the above-mentioned indication of the refractive error (more specifically, the degree of myopia/hyperopia) of the eye 12 to generate a second control signal for controlling the focussing mechanism 42 to focus the second light Lₛ₁ on the fundus 10 via the objective lens system 20 having its focal length set to the second focal length FL₂.

In the example of Figure 2, where the auxiliary optical system 40 takes the example form of a fixation target light projector, the controller 50 generates the second control signal for controlling the focussing mechanism 42 of the fixation target light projector to provide a focussing of the fixation target light so that, when the focussing mechanism 42 provides this focussing and the objective lens system 20 has had its focal length set to the second focal length FL₂, the projection of the fixation target on the fundus 10 is in focus on the fundus 10. The controller 50 may store (or access in an external memory) a mapping which associates values of the first and second indications with corresponding values of the second control signal, and use this mapping, together with the first or second indication acquired in process S10 of Figure 6, to generate the second control signal in process S30 of Figure 6. The mapping may be provided in any suitable form, for example one or more look-up tables (LUTs) or any suitable function(s) (e.g. a polynomial) that relate values of the first and second indication to corresponding values of the second control signal. The mapping may, for example, be determined by calibration, specifically by finding the degree of adjustment of the focussing mechanism 42 required to achieve an in-focus image of the fixation target on the retinas of subjects having varying degrees of myopia and hyperopia, when the focal length of the objective lens system 20 is set to the second focal length FL₂. The mappings may alternatively be calculated, using an optical model of the ophthalmic imaging instrument 100 and of an eye under examination having varying degrees of myopia and hyperopia. The optical model may be created in any suitable optical modelling software, such as Ansys Zemax OpticStudio^{™}, for example.

The subject therefore continues to see the fixation target as the above-described adjustments to the focussing mechanism 42 and the objective lens system 20 are being made to allow the ophthalmic imaging instrument 100 to switch from operating in the PS imaging mode to operate in the AS imaging mode, with little or no change in the degree of focus of the fixation target being perceived by the subject whilst the adjustments are being made. The fixation target is in focus on the fundus 10 after the adjustments have been completed, and therefore provides an effective aid for fixing the subject's gaze direction during the imaging of the anterior segment 14.

In the alternative example described above, where the auxiliary optical system 40 comprises a fundus tracking optical system 80 as illustrated schematically in Figure 3, the controller 50 generates the second control signal for controlling the focussing mechanism 42 to provide a focussing of the signal light L_{f1} so that, when the focussing mechanism 42 provides this focussing and the objective lens system 20 has had its focal length set to the second focal length FL₂, the image-forming device 82 is able to acquire images of the illuminated portion of the fundus 10, in which images the fundus 10 (e.g. the surface of the retina) is in focus. As the acquired fundus images are in focus, the processor 85 may acquire more reliable tracking data D by correlating the fundus images as described above, and the mentioned control loop may be able to start maintaining the position on the imaged part of the anterior segment 14 where the signal light L_{f1} is projected onto the imaged part as soon as the tracking data D is available, so that the anterior segment imaging can begin with little or no delay after the fundus 10 has been imaged.

Although the controller 50 preferably performs the above-described adjustments of the focal length of the objective lens system 20 to the second focal length FL₂ and of the focussing of the second light Lₛ₁ by the focussing mechanism 42 simultaneously, this need not be the case, and these adjustments made be made one after another in other example embodiments. The focussing mechanism 42 may therefore be controlled by the controller 50 to focus the second light Lₛ₁ such that, after the objective lens system 20 has previously or subsequently had its focal length set to the second focal length FL₂, the projection of the second light Lₛ₁ onto the fundus 10 is in focus.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the controller 50, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While this specification contains many specific embodiment details, these should not be construed as limitations on the scope of any inventions or of what may be claimed, but rather as descriptions of features specific to particular embodiments described herein.

Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A controller (50) arranged to generate control signals for controlling an ophthalmic imaging instrument (100) which is operable in a posterior segment imaging mode to image a fundus (10) of an eye (12) and in an anterior segment imaging mode to image an anterior segment (14) of the eye (12), the ophthalmic imaging instrument (100) comprising:
an objective lens system (20);
an optical imaging system (30; 30') comprising a light source (32; 32') and an image-forming device (34; 34'), the optical imaging system (30; 30') and the objective lens system (20) being arranged such that:
in the posterior segment imaging mode, the light source (32; 32') illuminates a portion of the fundus (10) with a first light (L_{f1}) via the objective lens system (20), and the image-forming device (34; 34') images the portion of the fundus (10) via the objective lens system (20); and
in the anterior segment imaging mode, the light source (32; 32') illuminates a portion of the anterior segment (14) of the eye (12) with the first light (L_{f1}) via the objective lens system (20), and the image-forming device (34; 34') images the portion of the anterior segment (14) via the objective lens system (20),
wherein the objective lens system (20) has a focal length which is adjustable over focal lengths including a first focal length (FL₁) for imaging the portion of the fundus (10) in the posterior segment imaging mode, and a second focal length (FL₂) for imaging the portion of the anterior segment (14) in the anterior segment imaging mode; and
an auxiliary optical system (40) arranged to project a second light (Lₛ₁) onto the fundus (10) via the objective lens system (20) in the posterior segment imaging mode and in the anterior segment imaging mode, the auxiliary optical system (40) comprising a focussing mechanism (42) which is controllable to vary a degree of focus of the second light (Lₛ₁) on the fundus (10),
the controller (50) comprising computer hardware arranged to:
acquire one of a first indication of a degree of myopia of the eye (12) or a second indication of a degree of hyperopia of the eye (12);
generate a first control signal for controlling the objective lens system (20) to set its focal length to the second focal length (FL₂); and
use the one of the first indication or the second indication to generate a second control signal for controlling the focussing mechanism (42) to focus the second light (Lₛ₁) on the fundus (10) via the objective lens system (20) having its focal length set to the second focal length (FL₂).

2. The controller (50) according to claim 1, wherein the computer hardware is arranged to:
determine the one of the first indication or the second indication by determining an amount of adjustment of the focal length of the objective lens system (20) required to reach the first focal length (FL₁) for imaging the portion of the fundus (10) in the posterior segment imaging mode; and
use the determined amount of adjustment to generate the second control signal.

3. The controller (50) according to claim 1 or claim 2, wherein
the objective lens system (20) comprises:
a first focussing optical element (23) and a first drive mechanism (24) arranged to move the first focussing optical element (23) into and out of an optical path (P) through the objective lens system (20); and
a second drive mechanism (27) and a second focussing optical element (28) whose position along the optical path (P) through the objective lens system (20) is adjustable by the second drive mechanism (27), and
the computer hardware is arranged to generate the first control signal for controlling the objective lens system (20) to adjust its focal length to the second focal length (FL₂) by:
generating a first drive control signal for controlling the first drive mechanism (24) to move the first focussing optical element (23) into the optical path (P) through the objective lens system (20); and
generating a second drive control signal for controlling the second drive mechanism (27) to move the second focussing optical element (28) to a position along the optical path (P) through the objective lens system (20) such that the focal length of the objective lens system (20) is the second focal length (FL₂) for imaging the anterior segment (14) of the eye (12).

4. An ophthalmic imaging instrument (100) operable in a posterior segment imaging mode to image a fundus (10) of an eye (12), and in an anterior segment imaging mode to image an anterior segment (14) of the eye (12), the ophthalmic imaging instrument (100) comprising:
an objective lens system (20);
an optical imaging system (30; 30') comprising a light source (32; 32') and an image-forming device (34; 34'), the optical imaging system (30; 30') and the objective lens system (20) being arranged such that:
in the posterior segment imaging mode, the light source (32; 32') illuminates a portion of the fundus (10) with a first light (L_{f1}) via the objective lens system (20), and the image-forming device (34; 34') images the portion of the fundus (10) via the objective lens system (20); and
in the anterior segment imaging mode, the light source (32; 32') illuminates a portion of the anterior segment (14) of the eye (12) with the first light (L_{f1}) via the objective lens system (20), and the image-forming device (34; 34') images the portion of the anterior segment (14) via the objective lens system (20),
wherein the objective lens system (20) has a focal length which is adjustable over focal lengths including a first focal length (FL₁) for imaging the portion of the fundus (10) in the posterior segment imaging mode, and a second focal length (FL₂) for imaging the portion of the anterior segment (14) in the anterior segment imaging mode;
an auxiliary optical system (40) arranged to project a second light (Lₛ₁) onto the fundus (10) via the objective lens system (20) in the posterior segment imaging mode and in the anterior segment imaging mode, the auxiliary optical system (40) comprising a focussing mechanism (42) which is controllable to vary a degree of focus of the second light (Lₛ₁) on the fundus (10); and
the controller (50) according to any preceding claim.

5. The ophthalmic imaging instrument (100) according to claim 4, wherein
the optical imaging system comprises an optical coherence tomography, OCT, imaging system (30') having a sample arm (31-1) along which the first light (L_{f1}) propagates to the eye (12) via the objective lens system (20), and a reference arm (31-2) along which a reference light (Lᵣ₂) from the light source (32') propagates, and
the OCT imaging system (30') and the objective lens system (20) are arranged such that:
in the posterior segment imaging mode, the image-forming device (34') generates an OCT image (60-1) of the portion of the fundus (10) by detecting an interference between the reference light (Lᵣ₂) and a portion (L_{f2}) of the first light reflected by the portion of the fundus (10); and
in the anterior segment imaging mode, the image-forming device (34') generates an OCT image (60-2) of the portion of the anterior segment (14) by detecting an interference between the reference light (Lᵣ₂) and a portion (L_{f2}) of the first light reflected by the portion of the anterior segment (14).

6. The ophthalmic imaging instrument (100) according to claim 5, wherein the image-forming device (34') is further arranged to generate OCT angiography, OCTA, data (70) of the portion of the fundus (10) by repeatedly detecting an interference between the reference light (Lᵣ₂) and the portion (L_{f2}) of the first light reflected by the portion of the fundus (10) in the posterior segment imaging mode.

7. The ophthalmic imaging instrument (100) according to any of claims 4 to 6, wherein the auxiliary optical system (40) comprises a fixation target light projector arranged to project, as the second light (Lₛ₁), a fixation target light onto the fundus (10) for fixing a gaze direction of the eye (12).

8. The ophthalmic imaging instrument (100) according to any of claims 4 to 6, wherein the auxiliary optical system (40) comprises a fundus tracking optical system arranged to generate tracking data (D) indicative of lateral movements of the fundus (10) relative to the ophthalmic imaging instrument (100) by acquiring a sequence of images of a portion of the fundus (10) illuminated by the second light (Lₛ₁), and correlating images of the sequence of images to generate the tracking data (D).

9. The ophthalmic imaging instrument (100) according to any of claims 4 to 8, wherein the focussing mechanism (42) comprises a focussing optical element (43) movable along an optical path of the second light (Lₛ₁) towards the fundus (10) so as to vary the degree of focus on the fundus (10) of the second light (Lₛ₁) projected onto the fundus (10).

10. A computer-implemented method of generating control signals for controlling an ophthalmic imaging instrument (100) which is operable in a posterior segment imaging mode to image a fundus (10) of an eye (12) and in an anterior segment imaging mode to image an anterior segment (14) of the eye (12), the ophthalmic imaging instrument (100) comprising:
an objective lens system (20);
an optical imaging system (30; 30') comprising a light source (32; 32') and an image-forming device (34; 34'), the optical imaging system (30; 30') and the objective lens system (20) being arranged such that:
in the posterior segment imaging mode, the light source (32; 32') illuminates a portion of the fundus (10) with a first light (L_{f1}) via the objective lens system (20), and the image-forming device (34; 34') images the portion of the fundus (10) via the objective lens system (20); and
in the anterior segment imaging mode, the light source (32; 32') illuminates a portion of the anterior segment (14) of the eye (12) with the first light (L_{f1}) via the objective lens system (20), and the image-forming device (34; 34') images the portion of the anterior segment (14) via the objective lens system (20),
wherein the objective lens system (20) has a focal length which is adjustable over focal lengths including a first focal length (FL₁) for imaging the portion of the fundus (10) in the posterior segment imaging mode, and a second focal length (FL₂) for imaging the portion of the anterior segment (14) in the anterior segment imaging mode; and
an auxiliary optical system (40) arranged to project a second light (Lₛ₁) onto the fundus (10) via the objective lens system (20) in the posterior segment imaging mode and in the anterior segment imaging mode, the auxiliary optical system (40) comprising a focussing mechanism (42) which is controllable to vary a degree of focus of the second light (Lₛ₁) on the fundus (10),
the computer-implemented method comprising:
acquiring (S10) one of a first indication of a degree of myopia of the eye (12) or a second indication of a degree of hyperopia of the eye (12);
generating (S20) a first control signal for controlling the objective lens system (20) to set its focal length to the second focal length (FL₂); and
using the one of the first indication or the second indication to generate (S30) a second control signal for controlling the focussing mechanism (42) to focus the second light (Lₛ₁) on the fundus (10) via the objective lens system (20) having its focal length set to the second focal length (FL₂).

11. The computer-implemented method according to claim 10, wherein
the one of the first indication or the second indication is acquired (S10) by determining an amount of adjustment of the focal length of the objective lens system (20) required to bring it to the first focal length (FL₁), and
the second control signal is generated (S30) using the determined amount of adjustment.

12. The computer-implemented method according to claim 10 or claim 11, wherein
the objective lens system (20) comprises:
a first focussing optical element (23) and a first drive mechanism (24) arranged to move the first focussing optical element (23) into and out of an optical path (P) through the objective lens system (20); and
a second drive mechanism (27) and a second focussing optical element (28) whose position along the optical path (P) through the objective lens system (20) is adjustable by the second drive mechanism (25), and
generating (S20) the first control signal for controlling the objective lens system (20) to adjust its focal length to the second focal length (FL₂) comprises:
generating (S22) a first drive control signal for controlling the first drive mechanism (24) to move the first focussing optical element (23) into the optical path (P) through the objective lens system (20); and
generating (S24) a second drive control signal for controlling the second drive mechanism (27) to move the second focussing optical element (28) to a position along the optical path (P) through the objective lens system (20) such that the focal length of the objective lens system (20) is the second focal length (FL₂) for imaging the anterior segment (14) of the eye (12).

13. A computer program (245) comprising computer-readable instructions that, when executed by a processor (220), cause the processor (220) to perform a method according to at least one of claims 10 to 12.

14. A computer-readable storage medium (250) storing the computer program (245) according to claim 13.
